# EUROPEAN PATENT SPECIFICATION

(11) **EP 4 338 835 B1**
(45) Date of publication and mention of the grant of the patent: **09.04.2025**
(21) Application number: 21942417.3
(22) Date of filing: 28.05.2021
(51) Int. Cl.: B01J 31/12, C07H 1/00, C07H 13/04, C07F 7/22

(54) **SUPPORTED ORGANOTIN CATALYST AND PREPARATION METHOD FOR SUCROSE-6-CARBOXYLATE**
ORGANOZINN-TRÄGERKATALYSATOR UND HERSTELLUNGSVERFAHREN FÜR SACCHAROSE-6-CARBOXYLAT
CATALYSEUR D'ORGANO-ÉTAIN SUPPORTÉ ET PROCÉDÉ DE PRÉPARATION DE SACCHAROSE-6-CARBOXYLATE

(43) Date of publication of application: 20.03.2024
(73) Proprietor: Anhui Jinhe Industrial Co., Ltd., Chuzhou, Anhui 239200 (CN)
(72) Inventor: ZHANG, Zhengsong, Chuzhou, Anhui 239200 (CN); YANG, Zhijian, Chuzhou, Anhui 239200 (CN); CHEN, Chaohui, Chuzhou, Anhui 239200 (CN); SHEN, Dongdong, Chuzhou, Anhui 239200 (CN)
(74) Representative: Ipside
(86) International application number: PCT/CN2021/096969
(87) International publication number: WO 2022/246846

(56) References cited:
- EP-A2- 1 669 360
- CN-A- 1 215 731
- CN-A- 1 714 935
- CN-A- 102 002 078
- CN-A- 104 098 617
- CN-A- 107 308 984
- CN-A- 113 195 099

## Description

### Technical Field

The present disclosure relates to the technical field of chemical manufacturing, and in particular, to a supported organotin catalyst and a preparation method for sucrose-6-carboxylate.

### Background of the Invention

Sucralose has the characteristics of low calories, high sweetness, pure sweetness and high safety, and is one of the most competitive sweeteners which are artificially synthesized so far.

Sucrose-6-acetate is a key intermediate for synthesizing sucralose by a monoester method, and at present, there are mainly three methods for the production process of sucrose-6-acetate: the first is an orthoacetate method, in which sucrose-6-acetate is generated by acetylation at 4-or 6-position first and then transposition from the 4-position to the 6-position; the second is an organotin method, in which sucrose is first reacted with an organotin compound catalyst to generate a sucrose organotin ester, and then reacted with an acylating agent to produce sucrose-6-acetate; and the third is an enzymatic catalysis method, in which sucrose-6-acetate is generated by acetylation at 6-site making use of the catalytic performance of an active enzyme. Compared with each other, the organotin monoester method has the advantages of simple process, mild conditions, high yield, catalyst circulation, and less three wastes, thus becoming the main production process currently.

Existing organotin compound catalysts mainly comprise dihydrocarbyl tin oxide, 1,3-dihydrocarbyloxy-1,1,3,3-tetra-(hydrocarbyl) distannoxane, etc. These catalysts are dissolved in a sucrose solution to participate in the reaction in the production process, a sucrose organotin ester is first generated, and then sucrose-6-acetate is generated under the action of an acylating agent. In order to recover the catalyst, water needs to be added later to quench the acylating agent , and then the organotin compound is extracted and recovered by using an organic solvent. In this way, the subsequent chlorination reaction is affected after water is added, and the subsequent chlorination reaction can only be carried out with several times of distillation for dehydration needed to achieve an anhydrous state. And the processes such as water addition for quenching, extraction and recovery, distillation for dehydration and the like need to consume a large amount of manpower, equipment and energy, which is very uneconomical. EP1669360 discloses a supported Stannate catalyst on Silica useful for transesterification reactions. CN102002078 discloses a method for preparing sucrose-6-acetate by transesterification using carboxylic anhydrides, using a monolithic structure catalyst using a solid disulfate as catalytically active species.

In addition, since the existing organotin compounds are distributed in light and heavy phases during extraction and recovery, which are difficult to be completely recycled, causing 1-5% of the catalyst to be lost each time along with cases such as material entrainment in the reaction process. Not only must a new catalyst be supplemented for each time of production, but also the catalyst entrained away will disadvantageously affect the subsequent chlorination reaction.

### Summary of the Invention

In view of the problems of complex recovery processes, high processing loss and high cost of the organotin catalyst used in the prior art, the present application provides a supported organotin catalyst and a preparation method for sucrose-6-carboxylate in order to overcome the above-mentioned problems.

In order to achieve the above purpose, the present application adopts the following technical solutions.

According to one aspect of the present application, a supported organotin catalyst is provided, the supported organotin catalyst is prepared by coupling an organotin functional group to an inorganic carrier, and the coupling structure of the organotin functional group is as follows: 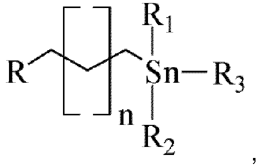 wherein, R is an atom of the inorganic carrier coupled to the organotin functional group; n is the number of carbon atoms of a carbon chain; R₁, R₂, and R₃ are groups on the tin atom, wherein at least one and at most two of the groups R₁, R₂, and R₃ are(is) hydrocarbyl, and the remainder(s) is (are) selected from one or two of an oxygen atom, a hydroxyl group, a hydrocarbyloxy group, and an acetoxy group.

Optionally, the atom R comprises one of silicon, oxygen, nitrogen, sulfur and phosphorus;
the number of carbon atoms of the carbon chain n is greater than or equal to 2;
the hydrocarbyl group is a linear or branched alkyl group, a cycloalkyl group, an aryl group or an aralkyl group, preferably a linear or branched alkyl group, and most preferably an n-butyl group; and the hydrocarbyloxy group is an alkoxy group or a phenoxy group, preferably a methoxy group, an ethoxy group, an n-propoxy group, an n-butoxy group, an n-pentyloxy group or an n-hexyloxy group, and most preferably a methoxy group.

Optionally, the inorganic carrier comprises one or more of silicon dioxide, titanium dioxide, activated carbon and aluminum oxide, preferably silicon dioxide; and
the inorganic carrier is prepared from a porous structure raw material having a specific surface area greater than 10 m²/g, preferably greater than 30 m²/g, and most preferably greater than 100 m²/g.

Optionally, in the supported organotin catalyst, the weight ratio of the loaded organotin functional group to the inorganic carrier is 1 ppm to 40%, preferably 1000 ppm to 10%.

Optionally, the organotin functional group and the inorganic carrier are coupled in the following manner:
forming an organotin catalyst with a silicon coupling atom by applying an organotin molecule having an organosiloxane or chlorosilane structure to the surface of silicon dioxide;
alternatively, forming an organotin catalyst with a nitrogen coupling atom by applying a halogenated organotin molecule to the surface of an inorganic carrier having an amino structure;
alternatively, forming an organotin catalyst with an oxygen coupling atom by applying a halogenated or hydroxyl organotin molecule to the surface of a carrier having a hydroxyl structure;
alternatively, forming an organotin catalyst with a sulfur coupling atom by applying a halogenated or hydroxyl organotin molecule to the surface of a carrier having a sulfhydryl structure;
or alternatively, forming an organotin catalyst with a phosphorus coupling atom by applying an organotin molecule having a phosphoric structure to the surface of an aluminum oxide carrier.

According to another aspect of the present application, a preparation method for sucrose-6-carboxylate is provided, and the method comprises:
a polar dissolution step: heating and dissolving sucrose in a polar aprotic solvent to obtain a sucrose solution;
a catalytic reaction step: adding the supported organotin catalyst as described in any one of the above to the sucrose solution for a suspension reaction;
a dehydration treatment step: dehydrating the reacted solution to obtain a suspension containing a sucrose organotin ester compound; and
an acylation reaction step: cooling the suspension of the sucrose organotin ester compound for reaction with a carboxylic anhydride acylating agent to obtain sucrose-6-carboxylate.

Optionally, in the polar dissolution step, the polar aprotic solvent used is selected from dimethyl sulfoxide, N-methyl pyrrolidone, N, N-dimethylacetamide, hexamethylphosphoramide and N, N-dimethylformamide, preferably N, N-dimethylformamide.

Optionally, in the polar dissolution step, the ratio of the volume usage of the polar aprotic solvent to the mass of sucrose is 2-50 L/kg, preferably 4-20 L/kg, and more preferably 6-10 L/kg.

Optionally, in the catalytic reaction step, the amount of the supported organotin catalyst is calculated according to the content of the organotin functional group, and the amount of the supported organotin catalyst is 0.01-2 molar equivalents based on sucrose, preferably 0.1-0.5 molar equivalent.

Optionally, in the dehydration treatment step, a non-polar solvent azeotropic dehydration method is used for dehydration treatment, and the non-polar solvent used is selected from cyclohexane, hexane, n-heptane, isooctane, benzene, toluene, chloroform, carbon tetrachloride, ethyl acetate and methyl acetate, preferably cyclohexane, hexane or n-heptane and most preferably cyclohexane; and the ratio of the addition amount of the non-polar solvent to the content of the polar aprotic solvent is less than 30%, preferably less than 20%, and most preferably less than 15%;
or alternatively, a single solvent distillation process is used for dehydration treatment with a distillation temperature of 40-100°C, preferably 60-80°C; the operating pressure is 0.01 kPa-100 kPa, preferably 0.5 kPa-90 kPa; and the distillation time is 1 minute to 12 hours, preferably 10 minutes to 2 hours.

Optionally, in the acylation reaction step, the carboxylic anhydride acylating agent used comprises acetic anhydride, butyric anhydride, benzoic anhydride, stearyl anhydride or lauric anhydride, and the obtained sucrose-6-carboxylate is respectively corresponding to sucrose-6-acetate, sucrose-6-butyrate, sucrose-6-benzoate, sucrose-6-fatty acid ester and sucrose-6-laurate; the acylation reaction temperature is 0-50°C, preferably 5-20°C; and the addition amount of the carboxylic anhydride acylating agent is 0.6-3.0 times of the amount of sucrose, preferably 0.8-1.5 times.

Optionally, the method further comprises:
an acylation quenching step: after the acylation reaction, adding water to quench the carboxylic anhydride acylating agent, the water addition amount is 30% or less of the total volume of the quenched solution, preferably 15% or less, and filtering and recovering the supported organotin catalyst after adding water.

In conclusion, the present application has the following beneficial effects:
The supported organotin catalyst of the present application is formed by coupling a specific organotin functional group to an inorganic carrier and can be used for catalyzing to generate sucrose-6-carboxylate, only a small amount of water needs to be added to quench the acylating agent after the reaction, and the supported organotin catalyst can be just directly filtered and recycled, so that the subsequent dehydration process is simpler and easier, and is very economical in terms of manpower, equipment and energy. Meanwhile, the organotin functional group is loaded on the inorganic carrier through a covalent bond of a coupling atom, so that the organotin functional group after filtration is hardly lost and is basically completely recycled, and there is not only no need to add a new supported organotin catalyst after each time of production, but also no bad influence on the subsequent chlorination reaction caused by the product being entrained with the supported organotin catalyst.

### Brief Description of the Drawings

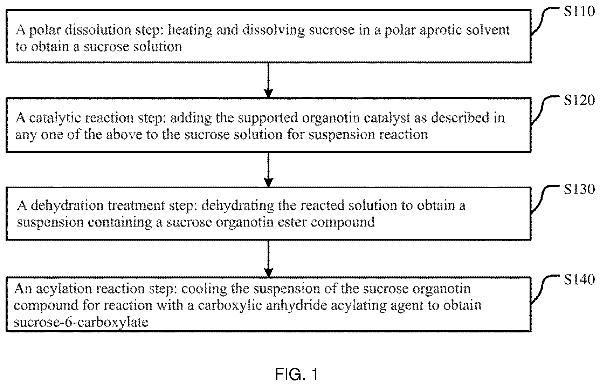

FIG. 1 is a schematic diagram of steps of a preparation method for sucrose-6-carboxylate according to an embodiment of the present application.

### Detailed Description of Embodiments

In order to make the objectives, technical solutions and advantages of the present disclosure clearer, the embodiments of the present disclosure will be further described in detail below with reference to the accompanying drawing.

The technical conception of the present application is that the supported organotin catalyst of the present application is formed by coupling a specific organotin functional group to an inorganic carrier and can be used for catalyzing to generate sucrose-6-carboxylate, only a small amount of water is needed to be added to quench the acylating agent after the reaction, and the supported organotin catalyst can be just directly filtered and recycled, so that the subsequent dehydration process is simpler and easier, and is very economical in terms of manpower, equipment and energy. Meanwhile, the organotin functional group is loaded on the inorganic carrier through a covalent bond of a coupling atom, so that the organotin functional group is hardly lost after filteration and is basically completely recycled, and therefore, there is not only no need to add a new supported organotin catalyst after each time of production, but also no bad influence on the subsequent chlorination reaction caused by the product being entrained with the supported organotin catalyst.

In one embodiment of the present application, the supported organotin catalyst is prepared by coupling an organotin functional group to an inorganic support, that is, by means of a certain manner, loading a specific organotin functional group onto the surface of an inorganic carrier by using a coupling atom on the inorganic carrier to form a solid catalyst having an organotin functional surface, these organotin functional surfaces being capable of catalyzing the reaction for sucrose-6-carboxylate, and because of the presence of the inorganic carrier, the supported organotin catalyst can conveniently participate in the reaction and be separated, recovered and reused with the reaction mother liquor. Wherein, the coupling structure of the organotin functional group is as follows: 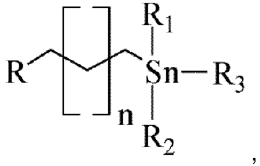 wherein, R is an atom of the inorganic carrier coupled to the organotin functional group; n is the number of carbon atoms of a carbon chain; R₁, R₂, and R₃ are groups on the tin atom, wherein at least one and at most two of the groups R₁, R₂, and R₃ are(is) hydrocarbyl, and the remainder(s) is (are) selected from one or two of an oxygen atom, a hydroxyl group, a hydrocarbyloxy group, and an acetoxy group. That is, when one of the groups R₁, R₂, and R₃ is a hydrocarbyl group, the other two are two of a hydroxyl group, a hydroxyl group and a hydrocarbyloxy group, or the other two are the same oxygen atom (i.e. the two groups thereof constitute an =O group); and when two of the groups R₁, R₂, and R₃ are hydrocarbyl groups, the other one is selected from one of a hydroxyl group, a hydrocarbyloxy group, and an acetoxy group.

The supported organotin catalyst of the embodiment of the present application is convenient to use, easy to filter, separate and recover, with no need to increase the amount of water like the current process to form two phases for extraction and recovery, so that the water addition amount for quenching the reaction is reduced, making the dehydration process faster and more efficient, and reducing the decomposition and side reactions of saccharide substances in the product system; and the supported organotin catalyst can reduce the content of unreacted sucrose by promoting the esterification reaction, thereby improving the selectivity and yield of the reaction.

Therefore, the supported organotin catalyst according to the embodiment of the present application can simplify the production process, achieve higher automated operation, reduce energy consumption and water consumption, reduce operating costs, and facilitate to realize a large-scale industrial production purpose.

In some embodiments of the present application, the atom R comprises one of silicon, oxygen, nitrogen, sulfur and phosphorus; the number of carbon atoms of the carbon chain n is greater than or equal to 2; the hydrocarbyl group is a linear or branched alkyl group, a cycloalkyl group, an aryl group or an aralkyl group, preferably a linear or branched alkyl group, and most preferably an n-butyl group; and the hydrocarbyloxy group is an alkoxy group or a phenoxy group, preferably a methoxy group, an ethoxy group, an n-propoxy group, an n-butoxy group, an n-pentyloxy group or an n-hexyloxy group, and most preferably a methoxy group.

In some embodiments of the present application, the inorganic carrier comprises one or more of silicon dioxide, titanium dioxide, activated carbon and aluminum oxide, preferably silicon dioxide.

In a further preferred embodiment, the inorganic carrier is prepared from a porous structure raw material, the inorganic carrier has a large specific surface area and can load a large number of organotin functional groups, and specifically, the specific surface area of the inorganic carrier is greater than 10 m²/g, preferably greater than 30 m²/g, and most preferably greater than 100 m²/g.

In some embodiments of the present application, in the supported organotin catalyst, the weight ratio of the loaded organotin functional group to the inorganic carrier is 1 ppm to 40%, preferably 1000 ppm to 10%.

In some embodiments of the present application, the loading of the organotin functional groups on the inorganic carrier involved may be performed by a person skilled in the art with reference to the prior art. Preferably, in some embodiments of the present application, considering the ease of coupling, and regarding different organotin molecular structures and inorganic carrier surface structures, the coupling between the organotin functional group and the inorganic carrier is achieved by selecting the following manner:
An organotin molecule having an organosiloxane or chlorosilane structure is applied to the surface of silicon dioxide to form an organotin catalyst with a silicon coupling atom. For example, a carrier having a silicon oxide surface (which may be silicon oxide, or other carriers coated with silicon oxide) is prepared as a suspension by using a solvent, and then an organotin molecule having an organosiloxane or chlorosilane structure is added to the suspension for a heat preservation reaction, so that covalent linkage is formed between the organosiloxane or the chlorosilane structure and the surface of the silicon oxide, thereby obtaining a solid catalyst loaded with an organotin functional group.

Alternatively, a halogenated organotin molecule is applied to the surface of an inorganic carrier having an amino structure to form an organotin catalyst with a nitrogen coupling atom. For example, an inorganic carrier having an aminated surface is suspended in a solvent to form a suspension, and then a halogen atom-substituted organotin molecule is added to the suspension for a heat preservation reaction, so that the amino group and the halogenated hydrocarbon structure react to form a covalent linkage, thereby obtaining a solid catalyst loaded with an organotin functional group.

Alternatively, a halogenated or hydroxyl organotin molecule is applied to the surface of an carrier having a hydroxyl structure to form an organotin catalyst with an oxygen coupling atom.

Alternatively, a halogenated or hydroxyl organotin molecule is applied to the surface of a carrier having a sulfhydryl structure to form an organotin catalyst with a sulfur coupling atom.

Alternatively, an organotin molecule having a phosphoric structure is applied to the surface of an aluminum oxide carrier to form an organotin catalyst with a phosphorus coupling atom.

By means of the above coupling method, a specific organotin functional group can be loaded onto an inorganic carrier by means of a covalent bond, thereby obtaining a solid catalyst having an organotin catalytic surface. Due to the existence of the inorganic carrier, the supported organotin catalyst according to the above embodiments of the present application can conveniently participate in the reaction and be separated, recovered and reused with the reaction mother liquor. In addition, since the supported organotin catalyst of the present application realizes loading by using a covalent bond, so that the structure and performance are stable, the organotin functional groups of the catalyst after filtration and recovery are hardly lost, therefore the complete recovery is basically realized, the supported organotin catalyst does not need to be supplemented after each time of production, and the problem that the subsequent chlorination reaction is affected by catalyst being incomplete recovered and entrained in a product system is also avoided.

The present application further discloses a preparation method for sucrose-6-carboxylate, FIG. 1 is a schematic embodiment of a preparation method for the sucrose-6-carboxylate, and the method comprises:
Step S110: a polar dissolution step: heating and dissolving sucrose in a polar aprotic solvent to obtain a sucrose solution.
Step S120: a catalytic reaction step: adding the supported organotin catalyst as described in any one of the above to the sucrose solution for a suspension reaction.
Step S130: a dehydration treatment step: dehydrating the reacted solution to obtain a suspension containing a sucrose organotin ester compound.

And step S140: an acylation reaction step: cooling the suspension of the sucrose organotin ester compound for reaction with a carboxylic anhydride acylating agent to obtain sucrose-6-carboxylate.

In some embodiments of the present application, in the polar dissolution step S110, the polar aprotic solvent used is selected from dimethyl sulfoxide, N-methylpyrrolidone, N, N-dimethylacetamide, hexamethylphosphoramide and N, N-dimethylformamide, preferably N, N-dimethylformamide.

In some embodiments of the present application, in the polar dissolution step S110, the ratio of the volume usage of the polar aprotic solvent to the mass of sucrose is 2-50 L/kg, preferably 4-20 L/kg, and more preferably 6-10 L/kg.

In some embodiments of the present application, in the catalytic reaction step S120, the amount of the supported organotin catalyst is calculated according to the content of the organotin functional group, and the amount of the supported organotin catalyst is 0.01-2 molar equivalents based on sucrose, preferably 0.1-0.5 molar equivalent.

In some embodiments of the present application, in the dehydration treatment step S130, a non-polar solvent azeotropic dehydration method is used for dehydration treatment, the non-polar solvent being used is selected from cyclohexane, hexane, n-heptane, isooctane, benzene, toluene, chloroform, carbon tetrachloride, ethyl acetate and methyl acetate, preferably cyclohexane, hexane or n-heptane, and most preferably cyclohexane, and the ratio of the addition amount of the non-polar solvent to the content of the polar aprotic solvent is less than 30%, preferably less than 20%, and most preferably less than 15%.

Or alternatively, in the dehydration treatment step S130, a single solvent distillation method is used for dehydration treatment with a distillation temperature of 40-100°C, preferably 60-80°C; the operating pressure is 0.01kPa-100kPa, preferably 0.5kPa-90kPa; and the distillation time is 1 minute -12 hours, preferably 10 minutes -2 hours.

In some embodiments of the present application, in the acylation reaction step S140, the carboxylic anhydride acylating agent used comprises acetic anhydride, butyric anhydride, benzoic anhydride, stearyl anhydride or lauric anhydride, and the obtained sucrose-6-carboxylate is respectively corresponding to sucrose-6-acetate, sucrose-6-butyrate, sucrose-6-benzoate, sucrose-6-fatty acid ester and sucrose-6-laurate; the acylation reaction temperature is 0-50°C, preferably 5-20°C; and the addition amount of the carboxylic anhydride acylating agent is 0.6-3.0 times of the amount of sucrose, preferably 0.8-1.5 times.

Wherein, the prepared sucrose-6-acetate and sucrose-6-benzoate can be used as intermediates for the synthesis of the sweetener sucralose, while other various sucrose-6-carboxylates can be used as synthetic intermediates for food additives, chemical products, and other reactions.

In an embodiment of the present application, the preparation method for sucrose-6-carboxylate further comprises: an acylation quenching step: after the acylation reaction, adding water to quench the carboxylic anhydride acylating agent, wherein the water adding amount is 30% or less of the total volume of the quenched solution, preferably 15% or less, and filtering and recovering the supported organotin catalyst after adding water. Compared with the prior art in which a large amount of water is added to carry out a separating phase extraction for recovery of the catalyst, the supported organotin catalyst of the present application is efficiently recovered by filtration after being used, so that the water addition amount is significantly reduced, distillation for dehydration are not needed, the decomposition and side reactions of carbohydrate substances in the product system in the distillation process are avoided, the process is also simplified, and the water consumption and energy consumption are reduced.

The following embodiments illustrate several preparation processes of the supported organotin catalyst of the present application, and the applying data of the supported organotin catalyst of each embodiment in the preparation process of sucrose-6-acetate.

Wherein, the content or purity of each substance in each embodiment is measured under the following conditions by using a High Performance Liquid Chromatography (HPLC) method, which will not be repeated in various embodiments. Analysis and determination conditions of high performance liquid chromatography: Japan Shimadzu High Performance Liquid Chromatograph, with RID-10A differential refraction detection, LC -10 ADVP high pressure pump, and CTO -10 ASVP incubator; chromatographic column: Agilent XDB C18 column (250 mm × 4.6 mm, 5 µm); mobile phase: methanol-0.125% dipotassium hydrogen phosphate aqueous solution (4: 6); column temperature: 30°C; and flow rate: 1.0 mL/min. Wherein, methanol (chromatographically pure), dipotassium hydrogen phosphate (analytically pure) and ultrapure water are needed, with external standard measurements used for detecting the content of each of standard substance.

### Embodiment 1

White carbon blackwith a high specific surface area (with a specific surface area of 200-450 m²/g) was taken, subjected to surface activation (hydrogen peroxide + sulfuric acid treatments) to form a hydrophilic surface, and dried for later use. The activated white carbon black (100 g) was taken and suspended in 1000 mL of toluene, and stirred under the protection of high purity nitrogen.

γ-trimethoxysilyldodecyl butyl tin oxide (20 g) was taken, dissolved in 100 mL of toluene, slowly added dropwise into the activated white carbon black suspension, reacted at 30-40 °C for 12 hours, evaporated under reduced pressure and dissolved to obtain 120 g of a supported organotin catalyst coupling by silicon atoms.

A suspension reaction solution was prepared at a ratio of sucrose: the supported organotin catalyst: DMF being 1: 2: 10, and heated to 80°C to be dissolved into a solution. A sucrose organotin ester compound was obtained after a qualified dehydration treatment using a bubble cap column reactor (the diameter of the bubble cap column in the lab was 50 cm, the size of the bubble cap was 30 × 8 mm, the number of plates was 5, and the retention time was about 5 minutes).

The obtained compound suspension contained 10% sugar by calculation, the obtained suspension was used as a reference, acetic anhydride was added dropwise at a ratio of acetic anhydride: the suspension being 1.1: 1 under the temperature condition of <10°C for acylation reaction, and after continuous reaction for 2 hours at the temperature of <10°C, a quenching reaction was performed with water at 0.05: 1 (water: suspension). The catalyst was centrifuged for filtration and recovered, the catalyst was washed with a small amount of DMF, the organic phase was combined, the obtained sucrose-6-acetate solution was analyzed by HPLC, and the products were as follows:
a. sucrose-6-acetate 10.30% (89.9% normalized);
b. diacetate 0.98% (8.6% normalized); and
c. sucrose 0.03% (0.24% normalized).

### Embodiment 2

White carbon black with a high specific surface area (with a specific surface area of 200-450 m²/g) was taken, subjected to surface activation (hydrogen peroxide + sulfuric acid treatments) to form a hydrophilic surface, and dried for later use. The activated white carbon black (100 g) was taken and suspended in 1000 mL of toluene, and stirred under the protection of high purity nitrogen. γ-aminotrimethoxysilane (10 g) was taken, dissolved with 100 mL of toluene, slowly added dropwise into the activated white carbon black suspension, reacted at 30-40°C for 12 hours, centrifuged for separation, washed with toluene and dried to obtain about 110 g of white carbon black optimized by amino on the surface.

The white carbon black optimized by amino on the surface (100 g) was taken and suspended in 1000 mL of toluene, and 20 g of γ-chlorohexyl dibutyl tin oxide was taken, dissolved in 100 mL of toluene, slowly added dropwise to the activated white carbon black suspension, reacted at 100-110°C for 12 hours, centrifuged for separation, washed with toluene and dried to obtain about 120 g of a supported organotin catalyst coupling by nitrogen atoms.

The supported organotin catalyst coupling by nitrogen atoms (50 g) and 200 mL cyclohexane were added into a 500 mL three-necked flask, stirred, heated to 40°C and stirred to form a suspension. Acetic anhydride (20 mL) was slowly added to the three-neck flask, and the addition was completed for about 30 minutes. After addition, the temperature was raised to 60°C to start heat preservation for dissolution for 3 hours. About 52 g of a supported organotin catalyst with acetoxy converted was obtained after centrifugal separation, washing and drying. The filtered mother liquor can be distilled under reduced pressure, and the solvent and a small amount of acetic anhydride contained therein was recovered.

The prepared supported organotin catalyst with acetoxy converted was used as a catalyst, 70 g of sucrose and 300 mL of DMF were added, and heated to 80°C. It was dehydrated using negative pressure, with a negative pressure of 101kPa and a temperature 70°C, anhydrous DMF was continuously supplemented until the water content in the distilled DMF solvent was 100 ppm or less, and it took about 2 hours.

The obtained solution contained about 20% sugar by calculation, the obtained solution was used as a reference, cyclohexane was added at 1: 2, acetic anhydride was added dropwise at a ratio of acetic anhydride: the solution being 1.1: 1 at a temperature of <10°C for acylation reaction, and after continuous reaction at the temperature of <10°C for 2 hours, the reaction was quenched with water at 0.05: 1 (water: solution). The supported organotin catalyst was centrifuged for separation, washed and recovered. The mother liquor was a sucrose-6-acetate solution, and the products were as follows by HPLC analysis:
a. sucrose-6-acetate 17.5% (89.9% normalized);
b. diacetate 1.68% (8.6% normalized); and
c. sucrose 0.05% (0.24% normalized).

### Embodiment 3

Aluminum oxide with a high specific surface area (with a specific surface area of 100-250 m²/g) was taken, subjected to surface activation (hydrogen peroxide treatment) to form a hydrophilic surface, and dried for later use. The activated aluminum oxide (100 g) was taken and suspended in 1000 mL of toluene, and stirred under the protection of high purity nitrogen.

γ- phosphoric hexyl dibutyl tin oxide (10 g) was taken, dissolved in 100 mL of toluene, slowly added dropwise into the activated aluminum oxide suspension, reacted at 100-110°C for 12 hours, centrifuged for separation, washed with toluene and dried to obtain about 120 g of a supported organotin catalyst coupling by phosphorus atoms.

The prepared supported organotin catalyst was used as a catalyst, added with 70 g of sucrose and 300 mL of DMF, and heated to 80°C to be dissolved into a solution. It was dehydrated using negative pressure, with a negative pressure of 101 kPa and a temperature of 65°C, anhydrous DMF was continuously supplemented until the water content in the distilled DMF solvent was 100 ppm or less, and it took about 2 hours.

The obtained solution contained 20% sugar by calculation, the obtained solution was used as a reference, cyclohexane was added at 1: 2, acetic anhydride was added dropwise at a ratio of acetic anhydride: the solution being 1.1: 1 at a temperature of <10°C for acylation reaction, and after continuous reaction at the temperature of <10°C for 2 hours, the reaction was quenched with water at 0.05: 1 (water: solution). The supported organotin catalyst was centrifuged for separation, washed and recovered. The mother liquor was a sucrose-6-acetate solution, and the products were as follows by HPLC analysis:
a. sucrose-6-acetate 17.83% (90.5% normalized);
b. diacetate 1.50% (7.6% normalized); and
c. sucrose 0.04% (0.21% normalized).

### Embodiment 4

White carbon black with a high specific surface area (with a specific surface area of 200-450 m²/g) was taken, subjected to surface activation (hydrogen peroxide + sulfuric acid treatments) to form a hydrophilic surface, and dried for later use. The activated white carbon black (100 g) was taken and suspended in 1000 mL of toluene, and stirred under the protection of high purity nitrogen. γ-mercaptotrimethoxysilane (10 g) was taken, dissolved in 100 mL of toluene, slowly added dropwise into the activated white carbon black suspension, reacted at 30-40 °C for 12 hours, centrifuged for separating, washed with toluene and dried to obtain about 110 g of white carbon black optimized by sulfydryl on the surface.

The white carbon black optimized by sulfydryl on the surface (100 g) was taken and suspended in 1000 mL of toluene, and 2 g of sodium metal was added for activation; 20 g of γ-chlorohexyl dibutyl tin oxide was taken, dissolved in 100 mL of toluene, slowly added dropwise to the activated white carbon black suspension, reacted at 100-110°C for 12 hours, centrifuged for separation, washed with toluene and dried to obtain about 120 g of a supported organotin catalyst coupling by sulfur atoms.

A 1000 mL four-mouth round-bottom flask was taken and equipped with mechanical stirring, a thermometer and a condenser tube with a water separator, 150 g of sucrose and 600 m1 of DMF were added, heated to 90°C for dissolution, cooled to 60°C, added with 120 g of the supported organotin catalyst coupling by sulfur atoms and 150 mL of cyclohexane, and heated to 90°C, and the amount of cyclohexane was adjusted for refluxing and separating water; the reaction was ended after 5 hours, and it was cooled to 5°C, added dropwise with 47.3 g of acetic anhydride which was completed in half an hour, heated to room temperature, and continuously stirred for 6 hours; and 5 mL of water was added, stirred for half an hour, centrifuged for separation, washed to recover the supported catalyst. The mother liquor was a DMF solution of sucrose-6-acetic acid, the high pressure liquid chromatography analysis shows that the purity of sucrose-6-ethyl in the solution is 95%, and the solution can be directly used for the next chlorination reaction.
a. sucrose-6-acetate 19.25% (87.5% normalization);
b. diacetate 2.46% (11.2% normalization); and
c. sucrose 0.08% (0.36% normalized).

### Embodiment 5

Macroporous activated carbon (with a specific surface of about 1000 m²/g) was taken and subjected to surface activation (hydrogen peroxide treatment) to form a hydrophilic surface, a layer of titanium dioxide with 10 nm thickness was coated on the surface of the activated carbon using a tetrabutyl titanate method, and it was centrifuged, washed and dried for later use. The activated carbon coated with titanium dioxide (100 g) was taken, suspended in 1000 mL of toluene, and stirred under the protection of high purity nitrogen. γ-hydroxytrimethoxy silane (10 g) was taken, dissolved in 100 mL of toluene, slowly added dropwise into the activated carbon suspension coated with titanium dioxide, reactd at 30-40°C for 12 hours, centrifuged for separation, washed with toluene and dried to obtain about 110 g of white carbon black optimized by hydroxyl on the surface.

The white carbon black optimized by hydroxyl on the surface (100 g) was taken, suspended in 1000 mL of toluene, and 2g of sodium metal was added for activation; 20 g of γ-chlorohexyl dibutyl tin oxide was taken, dissolved in 100 mL of toluene, slowly added dropwise into the activated white carbon black suspension, reactd at 100-110°C for 12 hours, centrifuged for separation, washed with toluene and dried to obtain about 120 g of a supported organotin catalyst coupling by oxygen atoms.

The prepared supported organotin catalyst coupling by oxygen atoms was used as a catalyst, added with 70g of sucrose and 300mL of DMF, and heated to 80°C to be dissolved into a solution. It was dehydrated using negative pressure, with a negative pressure of 95 kPa and a temperature of 75°C, anhydrous DMF was continuously supplemented until the water content in the distilled DMF solvent was 100 ppm or less, and it took about 4 hours.

The obtained solution contained 20% sugar by calculation, the obtained solution was used as a reference, cyclohexane was added at 1: 2, acetic anhydride was added dropwise at a ratio of acetic anhydride: the solution being 1.1: 1 at a temperature of <10°C for acylation reaction, and after continuous reaction at the temperature of <10°C for 2 hours, the reaction was quenched with water at 0.05: 1 (water: solution). The supported organotin catalyst was centrifuged for separation, washed and recovered. The mother liquor was a sucrose-6-acetate solution, and the products were as follows by HPLC analysis:
a. sucrose-6-acetate 16.264% (85.6% normalized);
b. diacetate 2.337% (12.3% normalized); and
c. sucrose 0.06% (0.31% normalized).

It can be seen from the data of the above embodiments that the supported organotin catalyst of the present application can effectively promote the esterification reaction and reduce the content of unreacted sucrose, thereby improving the selectivity and yield of the reaction, and the supported organotin catalysts of the embodiment of the present application are convenient to recycle, avoid the distillation and dehydration process, and also simplify the production process. That is, the supported organotin catalyst of the present application can improve the recovery efficiency of the supported organotin catalyst while improving the esterification reaction efficiency, and also simplify the process. It can be seen therefrom that the supported organotin catalyst of the present application can greatly improve the production efficiency, realize large-scale operation, and increase the operability of the process; the production process is simplified, realizing higher automatic operation; and the energy consumption is reduced, the operation cost is reduced, and the purpose of realizing large-scale industrial production is achieved.

The above are merely specific embodiments of the present disclosure, and under the above teachings of the present disclosure, a person skilled in the art may perform other improvements or modifications on the basis of the above embodiments. It should be understood by those skilled in the art that the above-mentioned specific description is merely a better explanation of the purpose of the present disclosure, and the scope of protection of the present disclosure shall be subject to the scope of protection of the claims.

## Claims

1. A supported organotin catalyst, wherein the supported organotin catalyst is prepared by coupling an organotin functional group to an inorganic carrier, and the coupling structure of the organotin functional group is as follows: 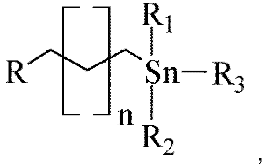 wherein, R is an atom of the inorganic carrier coupled to the organotin functional group; n is the number of carbon atoms of a carbon chain; R₁, R₂, and R₃ are groups on the tin atom, wherein at least one and at most two of the groups R₁, R₂, and R₃ are(is) hydrocarbyl, and the remainder(s) is(are) selected from one or two of an oxygen atom, a hydroxyl group, a hydrocarbyloxy group, and an acetoxy group.

2. The supported organotin catalyst according to claim 1, wherein the atom R comprises one of silicon, oxygen, nitrogen, sulfur and phosphorus;
the number of carbon atoms of the carbon chain n is greater than or equal to 2;
the hydrocarbyl group is a linear or branched alkyl group, a cycloalkyl group, an aryl group or an aralkyl group, preferably a linear or branched alkyl group, and most preferably an n-butyl group; and the hydrocarbyloxy group is an alkoxy group or a phenoxy group, preferably a methoxy group, an ethoxy group, an n-propoxy group, an n-butoxy group, an n-pentyloxy group or an n-hexyloxy group, and most preferably a methoxy group.

3. The supported organotin catalyst according to claim 1, wherein the inorganic carrier comprises one or more of silicon dioxide, titanium dioxide, activated carbon and aluminum oxide, preferably silicon dioxide; and
the inorganic carrier is prepared from a porous structure raw material, and the inorganic carrier has a specific surface area greater than 10 m2/g, preferably greater than 30 m2/g, and most preferably greater than 100 m2/g.

4. The supported organotin catalyst according to claim 1, wherein in the supported organotin catalyst, the weight ratio of the loaded organotin functional group to the inorganic carrier is 1 ppm -40%, preferably 1000 ppm -10%.

5. The supported organotin catalyst according to claim 1, wherein the organotin functional group and the inorganic carrier are coupling in the following manner:
forming an organotin catalyst with a silicon coupling atom by applying an organotin molecule having an organosiloxane or chlorosilane structure to the surface of silicon dioxide;
alternatively, forming an organotin catalyst with a nitrogen coupling atom by applying a halogenated organotin molecule to the surface of an inorganic carrier having an amino structure;
alternatively, forming an organotin catalyst with an oxygen coupling atom by applying a halogenated or hydroxyl organotin molecule to the surface of a carrier having a hydroxyl structure;
alternatively, forming an organotin catalyst with a sulfur coupling atom by applying a halogenated or hydroxyl organotin molecule to the surface of a carrier having a sulfhydryl structure;
or alternatively, forming an organotin catalyst with a phosphorus coupling atom by applying an organotin molecule having a phosphoric structure to the surface of an aluminum oxide carrier.

6. A preparation method for sucrose-6-carboxylate, wherein the method comprises:
a polar dissolution step: heating and dissolving sucrose in a polar aprotic solvent to obtain a sucrose solution;
a catalytic reaction step: adding the supported organotin catalyst according to claim 1 into the sucrose solution for a suspension reaction;
a dehydration treatment step: dehydrating the reacted solution to obtain a suspension containing a sucrose organotin ester compound; and
an acylation reaction step: cooling the suspension of the sucrose organotin ester compound for reaction with a carboxylic anhydride acylating agent to obtain sucrose-6-carboxylate.

7. The preparation method for sucrose-6-carboxylate according to claim 6, wherein in the step of polar dissolution, the polar aprotic solvent used is selected from dimethyl sulfoxide, N-methyl pyrrolidone, N, N-dimethylacetamide, hexamethylphosphoramide and N, N-dimethylformamide, preferably N, N-dimethylformamide.

8. The preparation method for sucrose-6-carboxylate according to claim 6, wherein in the polar dissolution step, the ratio of the volume usage of the polar aprotic solvent to the mass of sucrose is 2-50 L/kg, preferably 4-20 L/kg, and more preferably 6-10 L/kg.

9. The preparation method for sucrose-6-carboxylate according to claim 6, wherein in the catalytic reaction step, the amount of the supported organotin catalyst is calculated according to the content of the organotin functional group, and the amount of the supported organotin catalyst is 0.01-2 molar equivalents based on sucrose, preferably 0.1-0.5 molar equivalent.

10. The preparation method for sucrose-6-carboxylate according to claim 6, wherein in the dehydration treatment step, a non-polar solvent azeotropic dehydration method is used for dehydration treatment, wherein the non-polar solvent used is selected from cyclohexane, hexane, n-heptane, isooctane, benzene, toluene, chloroform, carbon tetrachloride, ethyl acetate and methyl acetate, preferably cyclohexane, hexane and n-heptane, and most preferably cyclohexane; and the ratio of the addition amount of the non-polar solvent to the content of the polar aprotic solvent is less than 30%, preferably less than 20%, and most preferably less than 15%;
or a single solvent distillation method is used for the dehydration treatment, and the distillation temperature is 40-100°C, preferably 60-80°C; the operating pressure is 0.01 kPa -100 kPa, preferably 0. 5 kPa -90 kPa; and the distillation time is 1 minute to 12 hours, preferably 10 minutes to 2 hours.

11. The preparation method for sucrose-6-carboxylate according to claim 6, wherein in the acylation reaction step, the carboxylic anhydride acylating agent used comprises: acetic anhydride, butyric anhydride, benzoic anhydride, stearyl anhydride or lauric anhydride, and the obtained sucrose-6-carboxylate is respectively corresponding to sucrose-6-acetate, sucrose-6-butyrate, sucrose-6-benzoate, sucrose-6-fatty acid ester and sucrose-6-laurate; the acylation reaction temperature is 0-50°C, preferably 5-20°C; and the addition amount of the carboxylic anhydride acylating agent is 0.6-3.0 times of the mass of sucrose, preferably 0.8-1.5 times.

12. The preparation method for sucrose-6-carboxylate according to claim 6, wherein the method further comprises:
an acylation quenching step: after the acylation reaction, adding water to quench the carboxylic anhydride acylating agent, wherein the addition amount of water is 30% or less of the total volume of the quenched solution, preferably 15% or less, and filtering and recovering the supported organotin catalyst after adding water.

## Patentansprüche

1. Gestützter Organotin-Katalysator, wobei der unterstützte Organotin-Katalysator durch Koppeln einer Organotin-Funktionsgruppe an einen anorganischen Träger vorbereitet wird, und die Koppelstruktur der Organotin-Funktionsgruppe wie folgt ist: 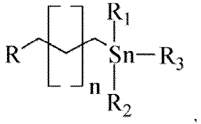
wobei R ein Atom des anorganischen Trägers ist, das mit der Organotin-Funktionsgruppe gekoppelt ist;
n ist die Anzahl der Kohlenstoffatome einer Kohlenstoffkette; R₁, R₂ und R₃ sind Gruppen auf dem Zinnatom, wobei mindestens ein und höchstens zwei der Gruppen R₁, R₂ und R₃ Hydrocarbyl sind(ist), und der/die Rest(e) aus einem oder zwei Sauerstoffatomen, einer Hydroxylgruppe, einer Hydrocarbyloxygruppe und einer Acetoxygruppe ausgewählt ist/sind.

2. Unterstützter Organotin-Katalysator nach Anspruch 1, wobei das Atom R eines der Elemente Silizium, Sauerstoff, Stickstoff, Schwefel und Phosphor umfasst;
die Anzahl der Kohlenstoffatome der Kohlenstoffkette n größer oder gleich 2 ist;
die Hydrocarbylgruppe eine lineare oder verzweigte Alkylgruppe, eine Cycloalkylgruppe, eine Arylgruppe oder eine Aralkylgruppe, vorzugsweise eine lineare oder verzweigte Alkylgruppe, und vorzugsweise eine n-Butylgruppe ist; und die Hydrocarbyloxygruppe eine Alkoxygruppe oder eine Phenoxygruppe, vorzugsweise eine Methoxygruppe, eine Ethoxygruppe, eine n-Propoxygruppe, eine n-Butoxygruppe, eine n-Pentyloxygruppe oder eine n-Hexyloxygruppe und vorzugsweise eine Methoxygruppe ist.

3. Unterstützter Organotin-Katalysator nach Anspruch 1, wobei der anorganische Träger einen oder mehrere der Elemente Siliziumdioxid, Titandioxid, Aktivkohle und Aluminiumoxid, vorzugsweise Siliziumdioxid, umfasst; und der anorganische Träger aus einem Rohmaterial mit poröser Struktur hergestellt ist, und der anorganische Träger eine spezifische Oberfläche von mehr als 10 m2/g, vorzugsweise von mehr als 30 m2/g und besonders vorzugsweise von mehr als 100 m2/g aufweist.

4. Gestützter Organotin-Katalysator nach Anspruch 1, wobei in dem unterstützten Organotin-Katalysator das Gewichtsverhältnis der geladenen Organotin-Funktionsgruppe zum anorganischen Träger 1 ppm -40 %, vorzugsweise 1000 ppm -10 % beträgt.

5. Unterstützter Organotin-Katalysator nach Anspruch 1, wobei die Organotin-Funktionsgruppe und der anorganische Träger wie folgt gekoppelt sind:
Bilden eines Organotin-Katalysators mit einem Siliziumkopplungsatom durch Aufbringen eines Organotin-Moleküls mit einer Organosiloxan- oder Chlorosilanstruktur auf die Oberfläche von Siliziumdioxid;
alternativ das Bilden eines Organotin-Katalysators mit einem Stickstoffkopplungsatom durch Aufbringen eines halogenierten Organotin-Moleküls auf die Oberfläche eines anorganischen Trägers mit einer Aminostruktur;
alternativ das Bilden eines Organotin-Katalysators mit einem Sauerstoffkopplungsatom durch Aufbringen eines halogenierten oder Hydroxylorganotin-Moleküls auf die Oberfläche eines Trägers mit einer Hydroxylstruktur;
alternativ das Bilden eines Organotin-Katalysators mit einem Schwefelkopplungsatom durch Aufbringen eines halogenierten oder Hydroxylorganotin-Moleküls auf die Oberfläche eines Trägers mit einer Sulfhydrylstruktur;
oder alternativ das Bilden eines Organotinkatalysators mit einem Phosphorkopplungsatom durch Aufbringen eines Organotin-Moleküls mit einer Phosphorstruktur auf die Oberfläche eines Aluminiumoxidträgers.

6. Zubereitungsverfahren für Saccharose-6-Carboxylat, wobei das Verfahren umfasst:
einen polaren Auflösungsschritt: Erhitzen und Auflösen von Saccharose in einem polaren aprotischen Lösungsmittel, um eine Saccharoselösung zu erhalten;
einen katalytischen Reaktionsschritt: Hinzufügen des unterstützten Organotin-Katalysators nach Anspruch 1 zur Saccharoselösung für eine Suspensionsreaktion;
einen Dehydrierungsbehandlungsschritt: Dehydrieren der reagierten Lösung, um eine Suspension zu erhalten, die eine Saccharose-Organotinester-Verbindung enthält; und
einen Acylierungsreaktionsschritt: Kühlen der Suspension der Saccharose-Organotinester-Verbindung zur Reaktion mit einem Acylierungsmittel aus Karboxylanhydrid, um Saccharose-6-Carboxylat zu erhalten.

7. Zubereitungsverfahren für Saccharose-6-Carboxylat nach Anspruch 6, wobei im Schritt der polaren Auflösung das verwendete polare aprotische Lösungsmittel aus Dimethylsulfoxid, N-Methylpyrrolidon, N, N-Dimethylacetamid, Hexamethylphosphoramid und N, N-Dimethylformamid, vorzugsweise N, N-Dimethylformamid ausgewählt wird.

8. Zubereitungsverfahren für Saccharose-6-Carboxylat nach Anspruch 6, wobei in dem Schritt der polaren Auflösung das Verhältnis der Volumennutzung des polaren aprotischen Lösungsmittels zur Masse der Saccharose 2-50 L/kg, vorzugsweise 4-20 L/kg und ganz vorzugsweise 6-10 L/kg beträgt.

9. Zubereitungsverfahren für Saccharose-6-Carboxylat nach Anspruch 6, wobei in dem katalytischen Reaktionsschritt die Menge des unterstützten Organotin-Katalysators entsprechend dem Gehalt der Organotin-Funktionsgruppe berechnet wird und die Menge des unterstützten Organotin-Katalysators 0,01-2 molare Äquivalente auf Basis von Saccharose, vorzugsweise 0,1-0,5 molare Äquivalente, beträgt.

10. Zubereitungsverfahren für Saccharose-6-Carboxylat nach Anspruch 6, wobei im Dehydrierungsbehandlungsschritt ein apolares azeotropes Lösungsmittel-Dehydrierungsverfahren für die Dehydrierungsbehandlung verwendet wird, wobei das verwendete apolare Lösungsmittel aus Cyclohexan, Hexan, n-Heptan, Isooctan, Benzol, Toluol, Chloroform, Kohlenstofftetrachlorid, Ethylacetat und Methylacetat, vorzugsweise Cyclohexan, Hexan und n-Heptan und besonders vorzugsweise Cyclohexan ausgewählt wird; und das Verhältnis der Zugabemenge des apolaren Lösungsmittels zum Gehalt des polaren aprotischen Lösungsmittels weniger als 30 %, vorzugsweise weniger als 20 % und besonders vorzugsweise weniger als 15 % beträgt;
oder ein Einzellösemitteldestillationsverfahren für die Dehydrierungsbehandlung verwendet wird, und die Destillationstemperatur 40-100 °C, vorzugsweise 60-80 °C beträgt; der Betriebsdruck 0,01 kPa -100 kPa, vorzugsweise 0,5 kPa-90 kPa beträgt; und die Destillationszeit 1 Minute bis 12 Stunden, vorzugsweise 10 Minuten bis 2 Stunden beträgt.

11. Zubereitungsverfahren für Saccharose-6-Carboxylat nach Anspruch 6, wobei im Acylierungsreaktionsschritt das verwendete Acylierungsmittel Karboxylanhydrid Folgendes umfasst: Essiganhydrid, Butyranhydrid, Benzoeanhydrid, Stearylanhydrid oder Laurinanhydrid, und das erhaltene Saccharose-6-Carboxylat jeweils Saccharose-6-Acetat, Saccharose-6-Butyrat, Saccharose-6-Benzoat, Saccharose-6-Fettsäureester und Saccharose-6-Laurat beträgt; die Acylationsreaktionstemperatur 0-50 °C, vorzugsweise 5-20 °C beträgt; und die Zugabemenge des Karboxylanhydrid-Acylierungsmittels das 0,6-3,0-Fache der Saccharosemasse, vorzugsweise das 0,8-1,5-Fache, beträgt.

12. Zubereitungsverfahren für Saccharose-6-Carboxylat nach Anspruch 6, wobei das Verfahren ferner umfasst:
einen Acylierungsabschreckschritt: nach der Acylierungsreaktion das Hinzufügen von Wasser zum Abschrecken des Karboxylanhydrid-Acylierungsmittels, wobei die Zugabemenge Wasser 30 % oder weniger des Gesamtvolumens der abgeschreckten Lösung, vorzugsweise 15 % oder weniger, beträgt, und das Filtern und Rückgewinnen des unterstützten Organotin-Katalysators nach dem Hinzufügen von Wasser.

## Revendications

1. Catalyseur à organo-étain supporté, dans lequel le catalyseur à organo-étain supporté est préparé en couplant un groupe fonctionnel organo-étain à un support inorganique, et la structure de couplage du groupe fonctionnel organo-étain est la suivante : 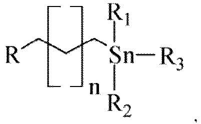
dans lequel, R est un atome du support inorganique couplé au groupe fonctionnel organo-étain ;
n est le nombre d'atomes de carbone d'une chaîne de carbones ; R₁, R₂, et R₃ sont des groupes sur l'atome d'étain, dans lequel au moins un et au plus deux des groupes R₁, R₂ et R₃ est (sont) un hydrocarbyle, et celui (ceux) qui reste(nt) est (sont) sélectionné(s) parmi un ou deux parmi un atome d'oxygène, un groupe hydroxyle, un groupe hydrocarbyloxy, et un groupe acétoxy.

2. Catalyseur à organo-étain supporté selon la revendication 1, dans lequel l'atome R comprend l'un parmi un silicium, un oxygène, un azote, un soufre et un phosphore ;
le nombre d'atomes de carbone de la chaîne de carbones, n, est supérieur ou égal à 2 ;
le groupe hydrocarbyle est un groupe alkyle linéaire ou ramifié, un groupe cycloalkyle, un groupe aryle ou un groupe aralkyle, de préférence un groupe alkyle linéaire ou ramifié, et de manière préférée entre toutes un groupe n-butyle ; et le groupe hydrocarbyloxy est un groupe alcoxy ou un groupe phénoxy, de préférence un groupe méthoxy, un groupe éthoxy, un groupe n-propoxy, un groupe n-butoxy, un groupe n-pentyloxy ou un groupe n-hexyloxy, et de manière préférée entre toutes un groupe méthoxy.

3. Catalyseur à organo-étain supporté selon la revendication 1, dans lequel le support inorganique comprend un ou plusieurs parmi le dioxyde de silicium, le dioxyde de titane, le charbon actif et l'oxyde d'aluminium, de préférence le dioxyde de silicium ; et
le support inorganique est préparé à partir d'une matière première à structure poreuse, et le support inorganique présente une surface spécifique supérieure à 10 m2/g, de préférence supérieure à 30 m2/g, et de manière préférée entre toutes supérieure à 100 m2/g.

4. Catalyseur à organo-étain supporté selon la revendication 1, dans lequel dans le catalyseur à organo-étain supporté, le rapport pondéral du groupe fonctionnel organo-étain chargé par rapport au support inorganique est de 1 ppm -40 %, de préférence de 1000 ppm -10 %.

5. Catalyseur à organo-étain supporté selon la revendication 1, dans lequel le groupe fonctionnel organo-étain et le support inorganique se couplent de la manière suivante :
la formation d'un catalyseur à organo-étain avec un atome de couplage silicium en appliquant une molécule d'organo-étain ayant une structure d'organosiloxane ou de chlorosilane sur la surface d'un dioxyde de silicium ;
en variante, la formation d'un catalyseur à organo-étain avec un atome de couplage azote en appliquant une molécule d'organo-étain halogénée sur la surface d'un support inorganique ayant une structure aminée ;
en variante, la formation d'un catalyseur à organo-étain avec un atome de couplage oxygène en appliquant une molécule d'organo-étain halogénée ou hydroxylée sur la surface d'un support ayant une structure hydroxylée ;
en variante, la formation d'un catalyseur à organo-étain avec un atome de couplage soufre en appliquant une molécule d'organo-étain halogénée ou hydroxylée sur la surface d'un support ayant une structure de sulfhydryle ;
ou en variante, la formation d'un catalyseur à organo-étain avec un atome de couplage phosphore en appliquant une molécule d'organo-étain ayant une structure phosphorique sur la surface d'un support de type oxyde d'aluminium.

6. Procédé de préparation de saccharose-6-carboxylate, dans lequel le procédé comprend :
une étape de dissolution polaire : chauffer et dissoudre du saccharose dans un solvant aprotique polaire pour obtenir une solution de saccharose ;
une étape de réaction catalytique : ajouter le catalyseur à organo-étain supporté selon la revendication 1 à la solution de saccharose pour une réaction en suspension ;
une étape de traitement de déshydratation : déshydrater la solution ayant réagi pour obtenir une suspension contenant un composé d'ester d'organo-étain de saccharose ; et
une étape de réaction d'acylation : refroidir la suspension du composé d'ester d'organo-étain de saccharose à des fins de réaction avec un agent d'acylation de type anhydride carboxylique pour obtenir du saccharose-6-carboxylate.

7. Procédé de préparation de saccharose-6-carboxylate selon la revendication 6, dans lequel, dans l'étape de dissolution polaire, le solvant aprotique polaire utilisé est sélectionné parmi le diméthylsulfoxyde, la N-méthylpyrrolidone, le N,N-diméthylacétamide, l'hexaméthylphosphoramide et le N,N-diméthylformamide, de préférence le N,N-diméthylformamide.

8. Procédé de préparation de saccharose-6-carboxylate selon la revendication 6, dans lequel, dans l'étape de dissolution polaire, le rapport de l'utilisation en volume du solvant aprotique polaire par rapport à la masse de saccharose est de 2 à 50 l/kg, de préférence de 4 à 20 l/kg, et de manière davantage préférée de 6 à 10 l/kg.

9. Procédé de préparation de saccharose-6-carboxylate selon la revendication 6, dans lequel, dans l'étape de réaction catalytique, la quantité du catalyseur à organo-étain supporté est calculée en fonction de la teneur en groupe fonctionnel organo-étain, et la quantité du catalyseur à organo-étain supporté est 0,01 à 2 équivalents molaires sur la base du saccharose, de préférence de 0,1 à 0,5 équivalent molaire.

10. Procédé de préparation de saccharose-6-carboxylate selon la revendication 6, dans lequel, dans l'étape de traitement de déshydratation, un procédé de déshydratation azéotrope par solvant non polaire est utilisé pour le traitement de déshydratation, dans lequel le solvant non polaire utilisé est sélectionné parmi le cyclohexane, l'hexane, le n-heptane, l'isooctane, le benzène, le toluène, le chloroforme, le tétrachlorure de carbone, l'acétate d'éthyle et l'acétate de méthyle, de préférence le cyclohexane, l'hexane et le n-heptane, et de manière préférée entre toutes le cyclohexane ; et le rapport de la quantité d'ajout du solvant non polaire par rapport à la teneur du solvant aprotique polaire est inférieur à 30 %, de préférence inférieur à 20 %, et de manière préférée entre toutes inférieur à 15 % ;
ou un procédé de distillation à solvant unique est utilisé pour le traitement de déshydratation, et la température de distillation est de 40 à 100 °C, de préférence de 60 à 80 °C ; la pression de fonctionnement est de 0,01 kPa à 100 kPa, de préférence de 0,5 kPa à 90 kPa ; et le temps de distillation est de 1 minute à 12 heures, de préférence de 10 minutes à 2 heures.

11. Procédé de préparation de saccharose-6-carboxylate selon la revendication 6, dans lequel, dans l'étape de réaction d'acylation, l'agent d'acylation de type anhydride carboxylique utilisé comprend : anhydride acétique, anhydride butyrique, anhydride benzoïque, anhydride stéarylique ou anhydride laurique, et le saccharose-6-carboxylate obtenu correspond respectivement au saccharose-6-acétate, au saccharose-6-butyrate, au saccharose-6-benzoate, à l'ester de saccharose-6-acide gras et au saccharose-6-laurate ; la température de réaction d'acylation est de 0 à 50 °C, de préférence de 5 à 20 °C ; et la quantité d'ajout de l'agent d'acylation de type anhydride carboxylique est de 0,6 à 3,0 fois la masse de saccharose, de préférence de 0,8 à 1,5 fois.

12. Procédé de préparation de saccharose-6-carboxylate selon la revendication 6, dans lequel le procédé comprend en outre :
une étape d'arrêt d'acylation : après la réaction d'acylation, ajouter de l'eau pour désactiver l'agent d'acylation de type anhydride carboxylique, dans lequel la quantité d'ajout d'eau est de 30 % ou moins du volume total de la solution désactivée, de préférence de 15 % ou moins, et filtrer et récupérer le catalyseur à organo-étain supporté après l'ajout d'eau.
